# EUROPEAN PATENT APPLICATION

(11) **EP 2 457 986 A1**
(43) Date of publication of application: **30.05.2012**
(21) Application number: 10193021.2
(22) Date of filing: 29.11.2010
(51) Int. Cl.: C11C 1/08, C11C 3/00, C10L 1/08

(54) **Production and separation of fatty acid alkyl esters**

(71) Applicant: Yellow Diesel B.V., 1018 WB Amsterdam (NL)
(72) Inventor: Dimian, Alexandre, C., 1183 EC Amstelveen (NL); Rothenberg, Gadi, 2341 NV Oestgeest (NL); Schut, Ronald, 1183 LP Amsterdam (NL)
(74) Representative: De Vries & Metman

(57) **Abstract**

Method and reactor system for separation of a mixture comprising an alcohol, fatty alkyl esters, glycerol and fatty acid glycerol esters. The fatty acid alkyl esters are separated from the mixture by membrane filtration, for example in two steps, wherein the mixture is first separated by membrane filtration into a first residue comprising at least a part of the fatty acid glycerol ester content, and an intermediate filtrate which is subsequently separated in a second membrane filtration step into a filtrate comprising at least a part of the alcohol content, and a second residue.

## Description

The present invention relates to a method and a reactor system for the production and separation of fatty acid alkyl esters, in particular for use as biodiesel.

Biodiesel can be produced by the transesterification of vegetable oil, such as soybean oil or rapeseed oil, and/or animal fats. Several methods are known for carrying out this transesterification process, such as batch processes, supercritical processes, ultrasonic methods, and microwave methods.

Transesterified biodiesel comprises a mix of mono-alkyl esters of long chain fatty acids. Typically, methanol is used for transesterification of the oil to produce methyl esters, generally referred to as Fatty Acid Methyl Ester (FAME), although ethanol can also be used to produce Fatty Acid Ethyl Esters (FAEE). Higher alcohols such as isopropanol and butanol have also been used. Homogenous or heterogeneous catalysts can be used to promote the transesterification process. Unlike the original vegetable oil these fatty acid alkyl esters, have combustion properties very similar to those of petroleum diesel. The fatty acid alkyl esters can be used as pure biodiesel or be blended with a petroleum diesel.

Although conversion rates can be obtained as high as 80 %, the resulting mixture still contains a considerable content of mono-, di- and triglycerides emulsified in a mixture of fatty acid alkyl esters, alcohol and glycerol.

It is an object of the invention to provide a method and a reactor system for the production of a biodiesel of improved purity with fewer yields of waste products.

The object of the invention is achieved with a method for separation of a mixture comprising an alcohol, fatty acid alkyl esters, glycerol and fatty acid glycerol esters, wherein the fatty acid alkyl esters are separated from the mixture by membrane filtration. Filtration processes are typically used for isolating larger particles from smaller ones. In this case, the fatty acid alkyl esters need to be isolated from alcohol as well as from the larger particles of the fatty acid glycerol esters. Notwithstanding this complication and notwithstanding the large variety in particle sizes of the various components in the mixture, it has surprisingly been found that membrane filtration is very effective in selective separation of the mixtures resulting from the transesterification of oils with alcohols.

The mixture can for instance first be separated in a first membrane filtration step into a first residue or retentate comprising at least a part of the fatty acid glycerol ester content, and an intermediate filtrate or permeate. In a second membrane filtration step the intermediate filtrate can then be separated into a filtrate or permeate comprising at least a part of the alcohol content, and a second residue or retentate. The second residue will mainly comprise a mixture of the fatty acid alkyl esters, glycerol and residual alcohol, which can be further processed to yield the desired biodiesel composition.

The first filtration step can for instance yield a residue with a fatty acid glycerol ester content of at least 80 wt%, e.g., of at least 90 wt.% or at least 95 wt.%, calculated on the total weight of the residue. The mixture will typically comprise mono-, di- and triglycerides. Optionally, this residue can be recycled to a transesterification process to enhance the overall conversion rate of the production process.

This first filtration step can be carried out using a microfiltration or ultrafiltration membrane. If the feedstock has a volumetric ratio methanol:triglyceride oil larger than 40% then a micro-emulsion can be formed with oil as dispersed phase. The size of the micelles is typically in the range 12 to 400 µm. Therefore, suitable pore size for the filter membrane would be in the range of 200 to 1000 nm. Without micro-emulsion the pore size of the membrane should be in the range 5 to 20 nm.

The filter membrane can be a hydrophilic or hydrophobic membrane, depending on the operating pressure, temperature and feedstock used. The membranes can for instance be made from polymeric materials, as polytetrafluoroethylene (PTFE), polypropylene, polyimide, polysulpholane, polycarbonate, etc., as well as from inorganic materials, such as porous ceramic, silica, α-alumina, microporous glass tubes, glass hollow fibres or mixtures thereof. Ceramic membranes offer higher chemical and thermal stability.

The second filtration step can for instance be carried out with a nano-filtration or pervaporation membrane, such as hybrid silica membranes. Suitable pervaporation membranes are commercially available, e.g., from Pervatech BV, the Netherlands, in particular the pervaporation membranes marketed under the trademark Hybsi®.

Generally, the used alcohol is at least partly methanol. In that case, the second membrane filter should be selective for methanol. To that end, the membrane filter should have an average pore diameter of 0,3 to 5 nm, e.g., 0.4 to 0.6 nm.

The alcohol content of the filtrate can for instance be as high as at least 80 wt.%, e.g., at least 90 wt.% or at least 95 wt.%, calculated on the total weight of the filtrate. Also this filtrate can be recycled to a transesterification process.

After the transesterification process, the temperature of the produced mixture can be as high as, e.g., 80°C. To increase the effectivity of the filtration steps, the mixture can for instance be cooled before passing the first membrane filtration step, for instance to a temperature of at most 50°C or at most 40°C or to an ambient temperature. The temperature can, e.g., be at least 5°C or at least 15°C.

Also homogenization of the mixture before passing the first membrane filtration step, e.g., by a static mixer, can contribute to the effectivity of the filtration.

The pressure in the mixture during filtration can be selected in such way that the alcohol is present in liquid form.

The obtained product is a mixture of glycerol, crude biodiesel and residual alcohol. Generally, the glycerol content of the mixture will be within the range of 8 to 10 wt.% and the fatty acid alkyl ester content will generally be within the range of 85 to 90 wt.% with some residual amounts of alcohol, the percentages by weight being calculated on total weight of the obtained product.

In this mixture a heavy phase with a high glycerol content can be separated more easily from a phase with a high fatty ester content by conventional techniques such as L-L decanting or centrifugation. The residual alcohol can be removed from one or both effluents by distillation to produce biodiesel and high purity glycerol.

The invention also pertains to a reactor system for the production of fatty acid alkyl esters comprising a transesterification station with an oil inlet, an alcohol inlet and an outlet, and a filtration unit comprising an retentate space, an permeate space and a filter membrane selective for at least one component in the mixture, the filter membrane separating the retentate space from the permeate space, wherein the retentate space is operatively connected to the outlet of the transesterification station and to a residue outlet, and wherein the permeate space comprises a filtrate outlet.

As used herein, "selective for at least one component" means that the filter membrane is configured to produce a filtrate comprising the largest part - e.g., at least 80 wt.% - of the component content in the mixture before filtration.

In a particularly suitable embodiment, the filter membrane is selective for fatty acid alkyl esters, glycerol and the used alcohol. The permeate space outlet can then be operatively connected to a second filtration unit comprising a second retentate space, a second permeate space and an alcohol selective filter membrane separating the second retentate space from the second permeate space. The second retentate space comprises an inlet and a second residue outlet and the second permeate space comprises a second filtrate outlet.

Optionally, a return line connects the residue outlet to the oil inlet of the transesterification station to recycle the fatty acid mono-, di- and triglycerides to improve the yield rate of the overall process and to reduce the amount of waste product.

Due to the high alcohol content of the second filtrate it can be reused in the transesterification process. To this end, a return line may connect the second filtrate outlet to the alcohol inlet of the transesterification station.

The invention also pertains to a filtration unit as such. In particular the invention pertians to a filtration unit comprising a first filter with a first retentate space, a first permeate space and a filter membrane selective for fatty acid alkyl esters, alcohol and glycerol, the first filter membrane separating the first retentate space from the first permeate space, wherein the first retentate space comprises a first inlet and a first residue outlet, wherein the first permeate space comprises a first filtrate outlet operatively connected to an inlet of a second filtration unit comprising a second retentate space connected to the inlet and a second residue outlet, a second permeate space with a second filtrate outlet and an alcohol selective filter membrane separating the second retentate space from the second permeate space.

The present invention will be elucidated with reference to the figures wherein:

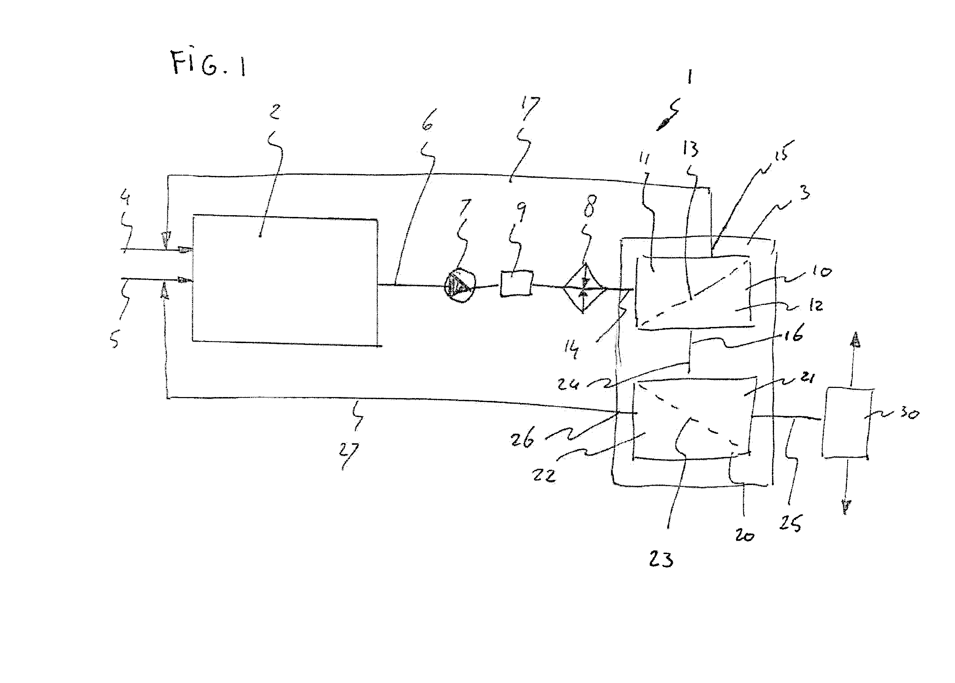

Figure 1: shows an embodiment of the process according to the invention in a flow diagram.

Figure 1 shows a flow diagram representing an embodiment of a reactor system 1 according to the present invention. The reactor system comprises a transesterification station 2 and a filtration unit 3. The transesterification station 2 comprises an oil inlet 4, an alcohol inlet 5 and an outlet 6 leading to the filtration unit 3. The outlet 6 is provided with a pump 7, a cooler 8 and a static mixer 9.

The filtration unit 3 comprises first filter 10 and a second filter 20. The first filter 10 comprises a retentate space 11, a permeate space 12 and a filter membrane 13 separating the retentate space 11 from the permeate space 12. the retentate space 11 has an inlet 14 which is operatively connected to the outlet 6 of the transesterification station 2. The retentate space 11 also comprises a residue outlet 15. The permeate space 12 comprises a filtrate outlet 16.

The filter membrane is selective for fatty acid alkyl esters and glycerol and has an average pore diameter of 1 micron.

The filtrate outlet 15 of the permeate space 12 is operatively connected to the second filter 20. Conform the first filter 10, the second filter comprises a second retentate space 21, a second permeate space 22 and an alcohol selective filter membrane 23 separating the second retentate space 21 from the second permeate space 22. The filter membrane 23 is alcohol selective and has an average pore diameter of 100 nm. The second retentate space 21 comprises an inlet 24 and a second residue outlet 25. The second permeate space 22 comprises a second filtrate outlet 26.

A first return line 17 connects the residue outlet 15 of the first filter 10 to the oil inlet 4 of the transesterification station 2. Likewise, a second return line 27 connects the second filtrate outlet 26 of the second filter 20 to the alcohol inlet 5 of the transesterification station 2.

A feedstock is supplied to the transesterification station 2. The feedstock comprises one or more oils and/or animal fats and one or more alcohols. These oils typically are triglycerides of C8 - C22 fatty acids. Suitable oils include for instance rapeseed oil, soybean oil, sunflower oil, palm oil, coconut oil, jatropha oil, karanja oil, cameline oil, algae oil, or waste vegetable oil (WVO). Suitable animal fats include tallow, lard, yellow grease, chicken fat, and the by-products of the production of omega-3 fatty acids from fish oil. Suitable alcohols include methanol, ethanol, butanol, propanol, iso-propanol or mixtures thereof.

Catalyst can be used for instance in the form of grains, extrudates or pellets of any form or size. Solid catalysts can, e.g., be basic or acidic or be a mixture of basic and acidic catalysts. Particular examples for acid catalysts are montmorillonite clays, heteropolyacids, sulphated and/or phosphated oxides, such as zirconia, titania, vanadia or niobia or mixed oxides. Particular examples of base catalysts are hydrotalcites, layered double hydroxides and solids comprised of basic functionalities, such as amino groups supported on carbon or on inorganic supports, such as silica, alumina and/or zirconia. Optionally, the catalysts can be mixed with one or more inert materials.

In the transesterification station 2 the mixture is subjected to a transesterification process to form a mixture of unreacted alcohol, fatty acid alkyl esters, glycerol and fatty acid glycerol esters. At this stage, the conversion rate is generally about 80 wt.%, e.g. at least 50 wt.% or at least 60 wt.%, and generally at most 90 wt.%. The mixture is typically an emulsion of particles containing fatty acid mono-, di- and triglycerides in a continuous phase of alcohol, glycerol and fatty acid alkyl esters.

Under the action of the pump 7 the mixture is transported via the outlet 6, the cooler 8, the static mixer 9, and the inlet of the filtration unit 10 into the retentate space 11. The mixture is separated into a residue comprising at least 70 wt.% of fatty acid mono-, di- and triglycerides, and a filtrate passing the membrane filter 13 to arrive in the permeate space 12. The filtrate comprises a mixture of fatty acid alkyl esters, glycerol and alcohol.

The residue comprising the major part of the fatty acid mono-, di- and triglycerides is passed via the residue outlet 15 and the return line 17 to the transesterification station 2. The filtrate comprising the mixture of fatty acid alkyl esters, glycerol and alcohol is transported to the second filter 20, where it enters the second retentate space 21. Most of the alcohol, e.g., at least 90 wt.% of the alcohol content of the filtrate of the first filter 10, will pass the alcohol selective membrane 23 to arrive in the permeate space 22 as a filtrate, which is recycled to the transesterification station 2 via the second filtrate outlet 26 and the second return line 27. A mixture of the fatty acid alkyl esters, glycerol and residual alcohol will leave the retentate space 21 via the residue outlet 25 to a further separation unit 30. The residual alcohol can be removed by distillation, and the glycerol is separated from the fatty acid alkyl esters forming a crude grade biodiesel.

## Claims

1. Method for separation of a mixture comprising an alcohol, fatty acid alkyl esters, glycerol and fatty acid glycerol esters, wherein at least a part of the fatty acid alkyl esters is separated from the mixture by membrane filtration.

2. Method according to claim 1 wherein the mixture is first separated by membrane filtration into a first residue comprising at least a part of the fatty acid glycerol ester content, and an intermediate filtrate which is subsequently separated in a second membrane filtration step into a filtrate comprising at least a part of the alcohol content, and a second residue.

3. Method according to claim 1 or 2 wherein the fatty acid glycerol ester content of the first residue is at least 80 wt% calculated on the total weight of the residue.

4. Method according to claim 2 or 3 wherein the first residue with the separated fatty acid glycerol ester content is recycled to a transesterification process.

5. Method according to claim 2, 3 or 4 wherein the alcohol content of the filtrate is at least 80 wt.%, calculated on the total weight of the filtrate.

6. Method according to any one of the preceding claims 2-5 wherein the mixture is made by transesterification of a feedstock with a volumetric ratio methanol:oil being larger than 35 %, e.g., larger than 40 %, and wherein the mixture is filtered by a filter membrane with an average pore size of at most 1 micron.

7. Method according to any one of the preceding claims 2 - 5 wherein the filtrate is recycled to a transesterification process.

8. Method according to any one of the preceding claims wherein the mixture is cooled and/or homogenized before passing the first membrane filtration step.

9. Reactor system (1) for the production of fatty acid alkyl esters comprising a transesterification station (2) with an oil inlet (4) , an alcohol inlet (5) and an outlet (6), and a filtration unit (3) comprising a filter (10) with an retentate space (11) with an inlet (14) operatively connected to the outlet of the transesterification station, an permeate space (12) and a filter membrane (13) selective for at least one component in the mixture, the filter membrane (13) separating the retentate space from the permeate space, wherein the retentate space comprises a residue outlet and the permeate space comprises a filtrate outlet (16).

10. Reactor system according to claim 9 wherein the filter membrane (13) is selective for fatty acid alkyl esters and glycerol, and wherein the filtrate outlet (16) is operatively connected to a second
filter (20) of the filtration unit (3), the second filter comprising a second retentate space (21), a second permeate space (22) and an alcohol selective filter membrane (23) separating the second retentate space from the second permeate space, wherein the second retentate space comprises an inlet (24) and a second residue outlet (25), wherein the second permeate space comprises a second filtrate outlet (26).

11. Reactor according to claim 10 wherein a return line (17) connects the residue outlet (15) to the oil inlet (4) of the transesterification station (2).

12. Reactor system according to claim 10 or 11 wherein a return line (27) connects the second filtrate outlet (26) to the alcohol inlet (5) of the transesterification station (2).

13. Reactor system according to claim 10, 11 or 12
wherein the alcohol selective filter membrane (23) is a methanol selective membrane with an average pore diameter within the range of 0.4 to 0.6 nm.

14. Reactor system according to any one of the preceding claims 10 - 13 wherein the filter membrane (13) selective for fatty acid alkyl esters and glycerol has an average pore diameter of at most 1 micron.

15. Filtration unit (3) comprising a first filter (10) with a first retentate space (11), a first permeate space (12) and a filter membrane (13) selective for
fatty acid alkyl esters and glycerol separating the first retentate space from the first permeate space, wherein the first retentate space comprises a first inlet (14) and a first residue outlet (15) and wherein the first permeate space comprises a first filtrate outlet (16) operatively connected to a second filter (20) of the filtration unit (3), the second filter comprising a second retentate space (21) with an inlet (24) and a second residue outlet (26), a second permeate space (22) with a second filtrate outlet (26) and an alcohol selective filter membrane (23) separating the second retentate space from the second permeate space.
